# EUROPÄISCHE PATENTSCHRIFT

(11) **EP 0 963 369 B1**
(45) Veröffentlichungstag und Bekanntmachung des Hinweises auf die Patenterteilung: **05.09.2001**
(21) Anmeldenummer: 98904088.6
(22) Anmeldetag: 17.01.1998
(51) Int. Cl.: C07C 253/34, C07C 255/24

(54) **VERFAHREN ZUR ABTRENNUNG VON 6-AMINOCAPRONITRIL AUS MISCHUNGEN, DIE 6-AMINOCAPRONITRIL UND EIN IMIN ENTHALTEN**
METHOD FOR SEPARATING 6-AMINOCAPRONITRILE FROM MIXTURES CONTAINING 6-AMINOCAPRONITRILE AND AN IMINE
PROCEDE POUR SEPARER DU 6-AMONICAPRONITRILE CONTENU DANS DES MELANGES COMPRENANT DU 6-AMINOCAPRONITRILE ET UNE IMINE

(30) Priorität: 07.02.1997 DE 19704620
(43) Veröffentlichungstag der Anmeldung: 15.12.1999
(73) Patentinhaber: BASF AKTIENGESELLSCHAFT, 67056 Ludwigshafen (DE)
(72) Erfinder: BASSLER, Peter, D-68519 Viernheim (DE); LUYKEN, Hermann, D-67069 Ludwigshafen (DE); REHFINGER, Alwin, D-67112 Mutterstadt (DE); RUST, Harald, D-67435 Neustadt (DE)
(86) Internationale Anmeldenummer: EP9800243
(87) Internationale Veröffentlichungsnummer: WO9834911

(56) Entgegenhaltungen:
- EP-A- 0 497 333
- US-A- 5 153 351
- US-A- 5 162 567

## Beschreibung

Die vorliegende Erfindung betrifft ein Verfahren zur destillativen Abtrennung von 6-Aminocapronitril aus Mischungen (I), die 6-Aminocapronitril und ein Imin (II) enthalten.

Die partielle Hydrierung von Adipodinitril zu 6-Amincapronitril in Gegenwart eines Katalysators auf der Basis eines Metalls wie Nickel, Cobalt, Eisen, Rhodium oder Ruthenium ist allgemein bekannt, beispielsweise aus EP-A-161 419, EP-A-77 911, US-A-4,389,348, US-A-4,601,859, WO 93/1207, DE-A 42 35 466, DE-A 19 500 222 und der Deutschen Anmeldung 19 548 289.1.

Als Nebenprodukte entstehen unter anderem Imine, insbesondere Tetrahydroazepin (THA) der Formel 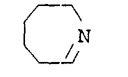 6-Aminocapronitril findet vor allem Verwendung für die Faserherstellung über Caprolactam als Zwischenstufe oder durch direkte Polymerisation zu Nylon 6. Hierzu muß das 6-Aminocapronitril eine hohe Reinheit aufweisen, wobei bekanntermaßen die Abtrennung von THA Probleme bereitet.

Aus US-A-5,162,567 ist bekannt, eine Mischung enthaltend 6-Aminocapronitril und THA mit einer organischen Carbonylverbindung, beispielsweise einem Keton oder einem Aldehyd, bei hoher Temperatur umzusetzen und anschließend 6-Aminocapronitril von der Mischung abzutrennen. Aus US-A-5,153,351 ist bekannt, eine Mischung enthaltend 6-Aminocapronitril und THA mit einer organischen C-H-aktiven Methylenverbindung, beispielsweise Malonitril, Cyclopentadien, Nitromethan oder Nitroethan, umzusetzen und an schließend 6-Aminocapronitril von der Mischung abzutrennen. Der Nachteil bei diesen Verfahren besteht darin, daß die Zugabe einer weiteren organischen Verbindung zu der Mischung den Aufwand bei der Reindarstellung von 6-Aminocapronitril erhöht.

Gemäß US-A-5,133,838 wird eine Mischung enthaltend 6-Aminocapronitril und THA mit einem anorganischen Hydrid wie Lithiumborhydrid umgesetzt. Nachteiligerweise muß bei diesem Verfahren das Hydrid in mehrfachem Überschuß der stöchiometrisch benötigten Menge eingesetzt werden. Zudem muß bei der anschließenden Destillation Sorge getragen werden, daß nicht das Wertprodukt 6-Aminocapronitril hydriert wird.

Die EP-A-497 333 beschreibt ein Verfahren, gemäß dem eine Mischung enthaltend 6-Aminocapronitril und THA mit einer alkalischen Verbindung umgesetzt wird. Nachteiligerweise muß die alkalische Verbindung im Überschuß der stöchiometrisch benötigten Menge eingesetzt und aus der erhaltenen Reaktionsmischung das 6-Aminocapronitril bei stark vermindertem Druck abdestilliert werden.

Aufgabe der vorliegenden Erfindung war es daher, ein Verfahren bereitzustellen, das die Abtrennung von 6-Aminocapronitril aus einer Mischung, die im wesentlichen 6-Aminocapronitril und THA enthält, auf technisch einfache und wirtschaftliche Weise unter Überwindung der genannten Nachteile bereitzustellen.

Demgemäß wurde ein Verfahren zur destillativen Abtrennung von 6-Aminocapronitril aus Mischungen (I), die 6-Aminocapronitril und ein Imin (II) enthalten, dadurch gekennzeichnet, daß man die Destillation in Gegenwart von Kohlendioxid durchführt, gefunden.

Mischungen (I) können in an sich bekannter Weise erhalten werden durch partielle Hydrierung von ADN , beispielsweise nach einem Verfahren gemäß EP-A-161 419, EP-A-77 911, US-A-4,389,348, US-A-4,601,859, WO 93/1207, DE-A 42 35 466, DE-A 19 500 222 und der Deutschen Anmeldung 19 548 289.1, indem man im allgemeinen die Hydrierung in Gegenwart von Nickel-, Cobalt-, Eisen-, Rhodium- oder Ruthenium-haltigen Katalysatoren durchführt. Dabei können die Katalysatoren als Trägerkatalysatoren oder als Vollkatalysatoren verwendet werden. Als Katalysatorträger kommen beispielsweise Aluminiumoxid, Siliciumdioxid, Titandioxid, Magnesiumoxid, Aktivkohlen und Spinelle in Frage. Als Vollkatalysatoren kommen beispielsweise Raney-Nickel und Raney-Cobalt in Betracht.

Bei der Hydrierung erhält man ein Gemisch, das 6-Aminocapronitril, HMD, ein Imin (II) und gegebenenfalls ADN enthält. Aus diesem Gemisch kann eine Mischung (I), die im wesentlichen 6-Aminocapronitril und ein Imin (II) enthält, beispielsweise durch Destillation erhalten werden.

Als Imin (II) kommen aromatische, vorzugsweise aliphatische, wie acyclische oder insbesondere cyclische Imine, sowie deren Gemi-5 sche, besonders bevorzugt THA in Betracht.

Die Imine (II) können in der Mischung (I) als einzelne Verbindungen oder als Addukte, beispielsweise an Amine wie 6-Aminocapronitril vorliegen, wobei diese Addukt eim Sinne der vorliegenden Erfindung ebenfalls als Imine (II) bezeichnet werden.

Kohlendioxid kann dem Destillationsgemisch vor oder vorzugsweise während der Destillation in Form einer Verbindung, die unter den Destillationsbedingungen Kohlendioxid freisetzt, wie Ammoniumcarbonat, Ammoniumcarbamat oder Harnstoff oder Gemische solcher Verbindungen, wobei diese Verbindungen in reiner Form oder in einem flüssigen Verdünnungsmittel, wie einem oder mehreren Bestandteilen der Mischung (I), zugegeben werden können, oder in Form von festem, flüssigem oder vorzugsweise gasförmigem Kohlendioxid, beispielsweise in Form eines Kohlendioxid enthaltenden Gases oder insbesondere in Form von reinem, nur die üblichen Verunreinigungen enthaltendem gasförmigem Kohlendioxid, erfolgen.

Der Kohlendioxid-Gehalt im Destillationsgemisch sollte vorteilhaft 0.1 bis 1 mol Kohlendioxid pro mol Iminfunktion des Imins (II) betragen.

In der Regel kommen dazu 0.022 bis 0.22 Nm³ pro kg Imin in Betracht.

Für die Destillation kommen hierfür übliche Apparaturen in Betracht, wie sie beispielsweise in: Kirk-Othmer, Encyclopedia of Chemical Technology, 3.Ed., Vol. 7, John Wiley & Sons, New York, 1979, Seite 870-881 beschrieben sind, wie Ventilbodenkolonnen, Siebbodenkolonnen, Füllkörperkolonnen oder Packungskolonnen.

Vorteilhaft kann man die Destillation im Bereich von 10 bis 100 mbar, bevorzugt bei 20 bis 50 mbar durchführen.

### Beispiele

6-Aminocapronitril mit den THA-Gehalten (Gew.-% bezogen auf die Mischung) gemäß Tabelle wurde in einer Destillationskolonne (Durchmesser 43 mm, Höhe 2.4 m, Gewebepackung Sulzer CY, 22 theoretische Trennstufen ) bei einem Rücklaufverhältnis von 5:1 diskontinuierlich fraktionierend destilliert. Das gereinigte 6-Aminocapronitril wurde als Destillat erhalten. Die Ergebnisse sind aus Tabelle 1 ersichtlich.

| | Bsp. 1 | Bsp. 2 | Bsp. 3 | Vgl.Bsp.1 | Vgl.Bsp.2 |
|---|---|---|---|---|---|
| THA-Gehalt im 6-Aminocapronitril % | 1,97 | 0,87 | 0,86 | 1,8 - | 1,81 |
| vorgelegtes Roh-6-Aminocapronitril in g | 1502 | 1487 | 1503 | 1440 | 1494 |
| Druck [mbar] | 20 | 20 | 20 | 50 | 20 |
| Sumpftemp. [°C] | 136 | 135 | 136 | 148 | 137 |
| Kohlendioxid-Zulauf NI/h | 5 | 8 | 10 | 0 | 0 |
| THA-Gehalt | | | | | |
| Destillat % | 0,7 | 0,27 | 0,24 | 1,3 | 1,4 |
| Abreichg. % | 66 | 69 | 72 | 28 | 22 |
| Sumpf % | 18 | 8,7 | 11,7 | 9,5 | 6,3 |
| Destillat in g | 1389 | 1454 | 1434 | 1246 | 1351 |
| Sumpf in g | 111 | 33 | 33 | 100 | 136 |

## Patentansprüche

1. Verfahren zur destillativen Abtrennung von 6-Aminocapronitril aus Mischungen (I), die 6-Aminocapronitril und ein Imin (II) enthalten, **dadurch gekennzeichnet**, daß man die Destillation in Gegenwart von Kohlendioxid durchführt.

2. Verfahren nach Anspruch 1, wobei man als Imin (II) ein zyklisches Imin einsetzt.

3. Verfahren nach Anspruch 1 oder 2, wobei man als Imin (II) Tetrahydroazepin einsetzt.

4. Verfahren nach den Ansprüchen 1 bis 3, wobei der Kohlendioxid-Gehalt im Destillationsgemisch 0,1 bis 1 mol Kohlendioxid pro mol Iminfunktion des Imins (II) beträgt.

5. Verfahren nach den Ansprüchen 1 bis 4, wobei man ein Kohlendioxid enthaltendes Gas in den Sumpf einer Destillationskolonne einleitet.

6. Verfahren nach den Ansprüchen 1 bis 5, wobei man die Destillation im Bereich von 10 bis 100 mbar durchführt, bevorzugt bei 20 bis 50 mbar durchführt.

7. Verfahren nach den Ansprüchen 1 bis 6, wobei man dem Destillationsgemisch eine Verbindung (III) zugibt, die unter den Destillationsbedingungen Kohlendioxid freisetzt.

8. Verfahren nach den Ansprüchen 1 bis 7, wobei man als Verbindung (III) Harnstoff, Ammoniumcarbonat, Ammoniumcarbamat und deren Gemische einsetzt.

## Claims

1. A process for distillative removal of 6-aminocapronitrile from mixtures (I) comprising 6-aminocapronitrile and an imine (II), which comprises conducting the distillation in the presence of carbon dioxide.

2. A process as claimed in claim 1, wherein imine (II) is a cyclic imine.

3. A process as claimed in claim 1 or 2, wherein imine (II) is tetrahydroazepine.

4. A process as claimed in any of claims 1 to 3, wherein the carbon dioxide content of the distillation mixture is within the range from 0.1 to 1 mol of carbon dioxide per mole of imine function of imine (II).

5. A process as claimed in any of claims 1 to 4, wherein a gas comprising carbon dioxide is introduced into the bottom region of a distillation column.

6. A process as claimed in any of claims 1 to 5, wherein the distillation is conducted within the range from 10 to 100 mbar, preferably within the range from 20 to 50 mbar.

7. A process as claimed in any of claims 1 to 6, wherein the distillation mixture has added to it a compound (III) which releases carbon dioxide under the distillation conditions.

8. A process as claimed in any of claims 1 to 7, wherein compound (III) is urea, ammonium carbonate, ammonium carbamate or a mixture thereof.

## Revendications

1. Procédé de séparation par distillation de 6-aminocapronitrile à partir de mélanges (I) contenant le 6-aminocapronitrile et une imine (II), **caractérisé en ce que** l'on entreprend la distillation en présence de dioxyde de carbone.

2. Procédé selon la revendication 1, où l'on met en oeuvre comme imine (II) une imine cyclique.

3. Procédé selon la revendication 1 ou 2, où l'on met en oeuvre comme imine (II) de la tétrahydroazépine.

4. Procédé selon les revendications 1 à 3, où la teneur en dioxyde de carbone du mélange de distillation est de 0,1 à 1 mole de dioxyde de carbone par mole de fonction imine de l'imine (II).

5. Procédé selon les revendications 1 à 4, où l'on introduit un gaz contenant du dioxyde de carbone dans le fond d'une colonne de distillation.

6. Procédé selon les revendications 1 à 5, où l'on entreprend la distillation dans la plage de 10 à 100 mbar, de préférence de 20 à 50 mbar.

7. Procédé selon les revendications 1 à 6, où l'on ajoute au mélange de distillation un composé (III), qui libère du dioxyde de carbone dans les conditions de la distillation.

8. Procédé selon les revendications 1 à 7, où l'on met en oeuvre en tant que composé (III) de l'urée, du carbonate d'ammonium, du carbamate d'ammonium et leurs mélanges.
